# FASCICULE DE BREVET EUROPEEN

(11) **EP 1 579 849 B1**
(45) Date de publication et mention de la délivrance du brevet: **05.08.2009**
(21) Numéro de dépôt: 05290410.9
(22) Date de dépôt: 18.03.2005
(51) Int. Cl.: A61Q 1/08, A61Q 1/10, A61Q 1/12, A61Q 17/04, A61Q 19/08, A61K 8/19, A61K 8/89

(54) **Compositions cosmétiques comprenant des particules concaves**
Kosmetische Zusammensetzungen enthaltend konkave Partikel
Cosmetic compositions comprising concave particles.

(30) Priorité: 22.03.2004 FR 0450560
(43) Date de publication de la demande: 28.09.2005
(73) Titulaire: L'ORÉAL, 75008 Paris (FR)
(72) Inventeur: Dumousseaux, Christophe, Sadohara cho Shinjukn Tokyo (JP)
(74) Mandataire: Boulard, Denis

(56) Documents cités:
- EP-A- 0 445 785
- US-B1- 6 461 595
- PATENT ABSTRACTS OF JAPAN vol. 2000, no. 10, 17 novembre 2000 (2000-11-17) & JP 2000 191789 A (TAKEMOTO OIL & FAT CO LTD), 11 juillet 2000 (2000-07-11)
- PATENT ABSTRACTS OF JAPAN vol. 2003, no. 09, 3 septembre 2003 (2003-09-03) & JP 2003 128788 A (TAKEMOTO OIL & FAT CO LTD), 8 mai 2003 (2003-05-08)

## Description

La présente invention a pour objet une composition cosmétique, en particulier sous forme de poudre compacte, comprenant des pigments et des particules concaves. L'invention a aussi pour objet un procédé cosmétique de maquillage ou de soin des matières kératiniques d'être humain, comme la peau, les cheveux, les ongles, et plus particulièrement la peau.

La composition selon l'invention peut être une composition de maquillage ou de soin de la peau et peut se présenter sous la forme d'un fard à joues, un fard à paupières, une poudre pour le visage, un fond de teint, un produit anticerne, un produit de maquillage du corps, d'un produit de soin du visage, d'un produit de soin du corps, d'un produit antisolaire. Plus spécialement, l'invention porte sur une composition de fond de teint.

Les poudres de maquillage comprennent généralement, d'une part, une phase pulvérulente comportant notamment des pigments et des charges et d'autre part, une phase grasse au titre de liant comprenant des corps gras, destinée à conférer au produit fini une certaine densité, à donner une douceur et une propriété émolliente au produit de maquillage et à favoriser son adhérence sur la peau.

Certaines compositions de maquillage telles que les fonds de teint, les fards à paupières ou les fards à joues se présentent sous forme de poudre compacte, comprenant généralement une phase grasse, appelée liant, et une phase pulvérulente comprenant notamment des pigments et/ou des charges.

L'élaboration des poudres compactes soulève de nombreuses difficultés car la composition finale doit être suffisamment homogène et compacte pour éviter une fragmentation du produit provoquée notamment par les chocs. La poudre compacte doit également présenter une bonne aptitude au prélèvement pour permettre à l'utilisatrice de prélever la quantité nécessaire de produit pour l'appliquer ensuite sur les matières kératiniques, notamment sur la peau.

Il est connu des demandes de brevet JP-A-2000-191789 et JP-A-2003-128788 des particules concaves de matériau d'organopolysiloxane réticulé qui ont la forme de portions de sphères creuses, obtenues par condensation de silanols résultant de l'hydrolyse de composés organosiliconés.
Dans ces demandes, il est suggéré de les utiliser notamment dans des produits cosmétique pour le visage ou dans des produits de maquillage, en particulier dans des poudres compactes de fond de teint. Ces produits de maquillage confèrent de la douceur lors de leur application sur la peau et adhèrent bien sur la peau. Ces demandes de brevet enseignent aussi de formuler ces particules dans des poudres compactes en présence de 16 % ou 20 % en poids d'huiles et 15 % en poids de dioxyde de titane ou de 25 % en poids de matières colorantes pulvérulentes. Or de telles poudres compactes ont l'inconvénient de présenter une mauvaise aptitude au prélèvement, elles se délitent difficilement au doigt ou à l'aide d'une éponge et l'utilisatrice ne parvient pas à prendre un quantité suffisante de produit pour pouvoir se maquiller convenablement. Cette difficulté à déliter le produit incite d'ailleurs souvent l'utilisatrice à frotter fortement sur la surface de la poudre compacte pour parvenir à prendre une quantité plus importante de produit mais le frottement plus important provoque un durcissement de la surface du produit rendant cette dernière lisse : le produit devient alors encore plus difficile à déliter. Par ailleurs, les propriétés de douceur et d'étalement sont difficilement perceptibles par l'utilisatrice lors de l'application du produit sur la peau.

La demande de brevet EP-A-0 445 785 décrit des compositions cosmétiques comprenant une phase grasse liquide et une phase pulvérulente comprenant des pigments et des particules à base de sulfate de baryum ayant une portion concave, lesdits pigments étant présents dans ces compositions en une teneur inférieure à 15% en poids, et la phase grasse liquide étant présente en une teneur inférieure à 13% en poids.

Le but de la présente invention est donc de disposer d'une composition cosmétique, notamment de maquillage, comprenant des particules concaves pouvant s'étaler facilement sur les matières kératiniques, en particulier sur la peau, et, lorsqu'elle se présente sous forme de poudre compacte, pouvant se déliter facilement au doigt ou à l'aide d'une éponge.

Les inventeurs ont découvert qu'une telle composition est obtenue en utilisant avec les particules concaves une teneur réduite en liant liquide et en pigments. La composition s'étale bien sur la peau, présente un bon glissant facilitant une bonne répartition du produit sur la surface de la peau et permettant ainsi d'obtenir un maquillage réparti de manière homogène sur la peau. De plus, lorsque la composition est sous forme de poudre compacte, cette dernière présente alors de bonnes propriétés de délitage permettant à l'utilisatrice de prendre facilement au doigt ou à l'éponge la quantité nécessaire de produit pour se maquiller.

De façon plus précise, l'invention a pour objet une composition cosmétique, notamment sous forme de poudre compacte, comprenant une phase pulvérulente et une phase grasse liquide, la phase pulvérulente comprenant des pigments et des particules concaves, sous forme de portions de sphères creuses constituées d'un matériau organosiliconé, les pigments étant présents en une teneur inférieure ou égale à 15 % en poids, par rapport au poids total de la composition, et la phase grasse liquide étant présente en une teneur inférieure ou égale à 13 % en poids, par rapport au poids total de la composition.

L'invention a également pour objet un procédé cosmétique de maquillage ou de soin non thérapeutique des matières kératiniques, notamment de la peau, comprenant l'application sur les matières kératiniques, notamment sur la peau, d'une composition telle que définie précédemment.

La composition selon l'invention comprend des particules concaves. Ces particules ont donc une surface présentant un arrondi intérieur.

Les particules concaves sont des particules de portions de sphères creuses constituées d'un matériau organosiliconé.

Lesdites particules ont avantageusement un diamètre moyen allant de 0,05 µm à 10 µm.

Les portions de sphères creuses utilisées dans la composition selon l'invention peuvent avoir la forme de sphères creuses tronquées, présentant un seul orifice communiquant avec leur cavité centrale, et ayant une section transversale en forme de fer à cheval ou d'arceau.

Le matériau organosiliconé est un polysiloxane réticulé de structure tridimensionnelle ; il comprend de préférence, voire est constitué de, des motifs de formule (I) : SiO₂ et de formule (II) : R¹SiO_{1,5}
dans lesquels R¹ désigne un groupe organique ayant un atome de carbone directement relié à l'atome de silicium. Le groupe organique peut être un groupe organique réactif, un groupe organique non réactif, et de préférence un groupe organique non réactif.

Le groupe organique non réactif peut être un groupe alkyle en C₁-C₄, notamment un groupe méthyle, éthyle, propyle, butyle, ou un groupe phényle, et de préférence un groupe méthyle.

Le groupe organique réactif peut être un groupe époxy, un groupe (méth)acryloxy, un groupe alkényle, un groupe mercaptoalkyle, aminoalkyle, halogénoalkyle, un groupe glycéroxy, un groupe uréïdo, un groupe cyano. De préférence, le groupe organique réactif peut être un groupe époxy, un groupe (méth)acryloxy, un groupe alkényle, un groupe mercaptoalkyle, aminoalkyle. Le groupe organique réactif comprend généralement de 2 à 6 atomes de carbone, notamment de 2 à 4 atomes de carbone.

Comme groupe époxy, on peut citer un groupe 2-glycidoxyéthyl, un groupe 3-glycidoxypropyl, un groupe 2-(3,4-époxycyclohexyl)propyl.
Comme groupe (méth)acryloxy, on peut citer un groupe 3-méthacryloxypropyl, un groupe 3-acryloxypropyl.
Comme groupe alkényle, on peut citer un groupe vinyl, allyl, isopropényl.
Comme groupe mercaptoalkyle, on peut citer un groupe mercaptopropyl, mercaptoéthyl.
Comme groupe aminoalkyle, on peut citer un groupe 3-(2-aminoéthyl)aminopropyl, un groupe 3-aminopropyl, un groupe N,N-diméthylaminopropyl.
Comme groupe halogénoalkyle, on peut citer un groupe 3-chloropropyl, un groupe trifluoropropyl.
Comme groupe glycéroxy, on peut citer un groupe 3-glycéroxypropyl, un groupe 2-glycéroxyéthyl.
Comme groupe uréido, on peut citer un groupe 2-uréidoéthyl.
Comme groupe cyano, on peut citer un groupe cyanopropyl, cyanoéthyl.

De préférence, dans le motif de formule (II), R¹ désigne un groupe méthyle.

Avantageusement, le matériau organosiliconé comprend les motifs (I) et (II) selon un rapport molaire motif (I) / motif (II) allant de 30/70 à 50/50, de préférence allant de 35/65 à 45/55.

Les particules d'organosilicone peuvent être notamment susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

L'étape (a) correspond à une réaction d'hydrolyse et l'étape (b) correspond à une réaction de condensation.

Dans l'étape (a), le rapport molaire du composé (III) au composé (IV) va habituellement de 30/70 à 50/50, avantageusement de 35/65 à 45/45, et est préférentiellement de 40/60. Le rapport en poids de l'eau au total des composés (III) et (IV) va de préférence de 10/90 à 70/30. L'ordre d'introduction des composés (III) et (IV) dépend généralement de leur vitesse d'hydrolyse. La température de la réaction d'hydrolyse va généralement de 0 à 40 °C et ne dépasse habituellement pas 30°C pour éviter une condensation prématurée des composés.

### Pour les groupements X et Y des composés (III) et (IV) :

Comme groupe alcoxy en C₁-C₄, on peut citer les groupes méthoxy, éthoxy ;
Comme groupe alkoxyéthoxy renfermant un groupe alcoxy en C₁-C₄, on peut citer les groupes méthoxyéthoxy, butoxyéthoxy ;
Comme groupe alcoxy en C₂-C₄, on peut citer les groupes acétoxy, propioxy ;
Comme groupe N,N-dilakylamino renfermant un groupement alkyle en C₁-C₄, on peut citer les groupes diméthylamino, diéthylamino ;
Comme atome d'halogène, on peut citer les atomes de chlore, de brome.

Comme composés de formule (III), on peut citer le tétraméthoxysilane, le tétraéthoxysilane, le tétrabutoxysilane, le triméthoxyéthoxysilane, le tributoxyéthoxysilane, le tétraacétoxysilane, le tétrapropioxysilane, le tétraacétoxysilane, le tétra(diméthylamino)silane, le tétra(diéthylamino)silane, le silane tétraol, le chlorosilane triol, le dichlorodisilanol, le tétrachlorosilane, le chlorotrihydrogénosilane. De préférence, le composé de formule (III) est choisi parmi le tétraméthoxysilane, le tétraéthoxysilane, le tétrabutoxysilane, et leurs mélanges.

Le composé de formule (III) conduit après la réaction de polymérisation à la formation des motifs de formule (I).

Le composé de formule (IV) conduit après la réaction de polymérisation à la formation des motifs de formule (II).

Le groupe R dans le composé de formule (IV) a la signification telle que décrite pour le groupe R¹ pour le composé de formule (II).

Comme exemple de composés de formule (IV) comportant un groupe R organique non réactif, on peut citer le méthyltriméthoxysilane, l'éthyltriéthoxysilane, le propyltributoxysilane, le butyltributoxysilane, le phényltriméthoxyéthoxysilane, le méthyl tributoxyethoxysilane, le méthyltriacétoxysilane, le méthyltripropioxysilane, le méthyltriacétoxysilane, le méthyltri(diméthylamino)silane, le méthyltri(diéthylamino)silane, le méthylsilane triol, le méthylchlorodisilanol, le méthyltrichlorosilane, le méthyltrihydrogénosilane.

Comme exemple de composés de formule (IV) comportant un groupe R organique réactif, on peut citer :
les silanes ayant un groupe époxy comme le 3-glycidoxypropyl triméthoxysilane, le 3-glycidoxypropyl triéthoxysilane, le 2-(3,4-époxycyclohexyl)éthyl triméthoxysilane, le 3-glycidoxypropylméthyl diméthoxysilane, le 3-glycidoxypropylméthyl diméthoxysilane, le 2-glycidoxyéthylméthyldiméthoxysilane, le 3-glycidoxypropyl diméthylméthoxysilane, le 2-glycidoxyéthyl diméthylméthoxysilane ;
les silanes ayant un groupe (méth)acryloxy comme le 3-méthacryloxypropyl triméthoxysilane, le 3-acryloxypropyl triméthoxysilane ;
les silanes ayant un groupe alkényle comme le vinyl triméthoxysilane, l'allyl triméthoxysilane, l'isopropényl triméthoxysilane ;
les silanes ayant un groupe mercapto comme le mercaptopropyl triméthoxysilane, le mercaptoéthyl triméthoxysilane ;
les silanes ayant un groupe aminoalkyle comme le 3-aminopropyl triméthoxysilane, le 3-(2-aminoéthyl)aminopropyl triméthoxysilane, le N,N-diméthylaminopropyl triméthoxysilane, le N,N-diméthylaminoéthyl triméthoxysilane ;
les silanes ayant un groupe halogénoalkyl comme le 3-chloropropyl triméthoxysilane, le trifluoropropyl triméthoxysilane ;
les silanes ayant un groupe glycéroxy comme le 3-glycéroxypropyl triméthoxysilane, le di(3-glycéroxypropyl)diméthoxysilane ;
les silanes ayant un groupe uréido comme le 3-uréïdopropyl triméthoxysilane, le 3-uréïdopropyl méthyldiméthoxysilane, le 3-uréïdopropyl diméthylméthoxysilane ;
les silanes ayant un groupe cyano comme le cyanopropyl triméthoxysilane, le cyanopropyl méthyldiméthoxysilane, le cyanopropyl diméthylméthoxysilane.

De préférence, le composé de formule (IV) comportant un groupe R organique réactif est choisi parmi les silanes ayant un groupe époxy, les silanes ayant un groupe (méth)acryloxy, les silanes ayant un groupe alkényle, les silanes ayant un groupe mercapto, les silanes ayant un groupe aminoalkyle.

Des exemples de composés (III) et (IV) préférés pour la mise en oeuvre de cette invention sont respectivement le tétraéthoxysilane et le méthyltriméthoxysilane.

Comme catalyseurs d'hydrolyse et de polymérisation, on peut utiliser indépendamment des catalyseurs basiques - tels que l'hydroxyde de sodium, l'hydroxyde de potassium, le carbonate de sodium, l'hydrogénocarbonate de sodium, ou des amines (telles qu'ammoniaque, triméthylamine, triéthylamine, hydroxyde de tétraméthylammonium) - ou des catalyseurs acides, choisis parmi les acides organiques - tels que l'acide citrique, l'acide acétique, l'acide méthanesulfonique, l'acide p-toulène sulfonique, l'acide dodécylbenzènesulfonique, l'acide dodécylsuifonique - ou minéraux - tels que l'acide chlorhydrique, l'acide sulfurique, l'acide phosphorique. Lorsqu'il est présent, le tensioactif utilisé est de préférence un tensioactif non ionique ou anionique ou un mélange des deux. Le dodécylbenzènesulfonate de sodium peut être utilisé comme tensioactif anionique. La fin de l'hydrolyse est marquée par la disparition des produits (III) et (IV), insolubles dans l'eau, et l'obtention d'une couche liquide homogène.

L'étape (b) de condensation peut utiliser le même catalyseur que l'étape d'hydrolyse ou un autre catalyseur choisi parmi ceux mentionnés précédemment.

A l'issue de ce procédé, on obtient une suspension dans l'eau de fines particules organosiliconées qui peuvent éventuellement être ensuite séparées de leur milieu. Le procédé décrit ci-dessus peut donc comprendre une étape supplémentaire de filtration, par exemple sur filtre à membrane, du produit résultant de l'étape (b), suivie éventuellement d'une étape de centrifugation du filtrat destinée à séparer les particules du milieu liquide, puis d'une étape de séchage des particules. D'autres méthodes de séparation peuvent bien entendu être employées.

Les particules obtenues (ou les sphères) ont de préférence un diamètre moyen allant de 0,05 à 10 µm.

La forme des portions de sphères creuses obtenues selon le procédé ci-dessus, ainsi que leurs dimensions, dépendront notamment du mode de mise en contact des produits à l'étape (b).

Un pH plutôt basique et une introduction à froid du catalyseur de polymérisation dans le mélange issu de l'étape (a) conduira à des portions de sphères creuses en forme de "bols" à fond arrondi, tandis qu'un pH plutôt acide, et une introduction goutte-à-goutte du mélange issu de l'étape (a) dans le catalyseur de polymérisation chaud, conduira à des portions de sphères creuses ayant une section transversale en forme de "fer à cheval".

Selon un mode de réalisation préféré de l'invention, on utilise des portions de sphères creuses en forme de "bols". Celles-ci peuvent être obtenues comme décrit dans la demande JP-2003 128 788.
Des portions de sphères creuses en forme de fer à cheval sont aussi décrites dans la demande JP-A-2000-191789.

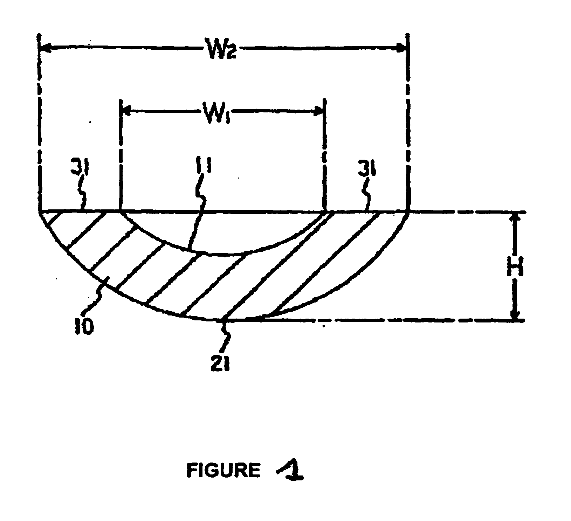

La figure annexée illustre une particule concave de portions de sphères en forme de bol en coupe transversale.

Comme il ressort de cette figure, ces portions concaves sont formées (en coupe longitudinale) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.

Les dimensions mentionnées ci-dessus sont obtenues en calculant la moyenne des dimensions de cent particules choisies sur une image obtenue au microscope électronique à balayage.

Comme particules concaves de portions de sphères utilisables selon l'invention, on peut citer :
les particules constituées de l'organosilicone réticulé TAK-110 (polymère réticulé méthylsilanol/silicate) de la société TAKEMOTO OIL & FAT , en forme de bol , de largeur 2,5 µm, de hauteur 1,2 µm et d'épaisseur 150 nm (particules vendues sous la dénomination NLK-506 par la société Takemoto Oil & Fat) ;
les particules constituées de l'organosilicone réticulé TAK-110 (polymère réticulé méthylsilanol/silicate) de la société TAKEMOTO OIL & FAT , en forme de bol , de largeur 2,5 µm, de hauteur 1,5 µm et d'épaisseur 350 nm ;
les particules constituées de l'organosilicone réticulé TAK-110 (polymère réticulé méthylsilanol/silicate) de la société TAKEMOTO OIL & FAT , en forme de bol , de largeur 0,7 µm, de hauteur 0,35 µm et d'épaisseur 100 nm ;
les particules constituées de l'organosilicone réticulé TAK-110 (polymère réticulé méthylsilanol/silicate) de la société TAKEMOTO OIL & FAT , en forme de bol , de largeur 7,5 µm, de hauteur 3,5 µm et d'épaisseur 200 nm.

Les particules concaves, notamment les particules de portions de sphères creuses, peuvent être présentes dans la composition selon l'invention, notamment dans la poudre compacte, en une teneur allant de 0,01 % à 50 % en poids, par rapport au poids total de la composition, de préférence allant de 0,1 % à 30 % en poids, et préférentiellement allant de 1 % à 15 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Les pigments peuvent également être choisies parmi les nanopigments d'oxydes métalliques, tels que le dioxyde de titane, l'oxyde de zinc, l'oxyde de fer, l'oxyde de zirconium, l'oxyde de cérium, et leurs mélanges.
Par nanopigments, on entend des pigments ayant une taille moyenne de particules allant de 1 nm à 500 nm, et de préférence allant de 10 nm à 100 nm.

Parmi les pigments organiques, on peut citer le noir de carbone, les pigments de type D & C, et les laques, notamment les laques à base de carmin de cochenille, de baryum, strontium, calcium, aluminium.

Les pigments peuvent être présents dans la composition, en un teneur allant de 0,1 % à 14,95 % en poids, par rapport au poids total de la composition, de préférence allant de 0,5 % à 12 % en poids, et préférentiellement allant de 1 % à 10 % en poids.

La poudre compacte selon l'invention peut comprendre une matière colorante pulvérulente additionnelle, différente des pigments décrits précédemment, et pouvant notamment être choisie parmi les nacres, les paillettes et leurs mélanges.

Les nacres peuvent être choisies parmi les nacres blanches telles que le mica recouvert de titane, ou d'oxychlorure de bismuth, les nacrés colorées telles que le mica titane recouvert avec des oxydes de fer, le mica titane recouvert avec notamment du bleu ferrique ou de l'oxyde de chrome, le mica titane recouvert avec un pigment organique du type précité ainsi que les nacres à base d'oxychlorure de bismuth.

Les nacres peuvent être présentes dans la composition en une teneur allant de 0,1 % à 50 % en poids, par rapport au poids total de la composition, de préférence allant de 0,1 % à 40 % en poids, et préférentiellement allant de 0,1 % à 30 % en poids.

La phase grasse de la poudre compacte, appelée généralement liant, est une phase grasse liquide à la température ambiante (25 °C) et peut comprendre une huile utilisée habituellement dans les poudres compactes.
L'huile peut être choisie parmi les huiles utilisées classiquement comme liant dans les poudres compactes. Parmi les huiles additionnelles utilisables, on peut citer l'huile de vison, l'huile de tortue, l'huile de soja, l'huile de pépins de raison, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide; les huiles d'hydrocarbures, telles que les huiles de paraffine, le squalane, la vaseline, le polydécène ; les esters gras, tels que le myristate d'isopropyle, le palmitate d'isopropyle, le stéarate de butyle, le stéarate d'isodécyle, le laurate d'hexyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine; les huiles de silicone telles que les polyméthylsiloxanes, les polyméthylphénylsiloxanes, les polysiloxanes modifiés par des acides gras, des alcools gras ou des polyoxyalkylènes, les silicones fluorées, les huiles perfluorées; l'acide oléique, l'acide linoléique, l'acide linolénique ; les alcools gras supérieurs tels que le cétanol ou l'alcool oléique.

La phase grasse liquide peut être présente en une teneur allant de 0,1 % à 13 % en poids, par rapport au poids total de la composition, de préférence allant de 0,1 % à 10 % en poids, et préférentiellement allant de 0,1 % à 8 % en poids.

La composition selon l'invention, notamment la poudre compacte, peut également comprendre des charges.
Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique ( par exemple feuillet, cubique, hexagonale, orthorombique, etc) . On peut citer le talc, le mica, la silice, le kaolin, les poudres de polyamide (Nylon®) , de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthanes, les charges composites, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les charges peuvent être présentes dans la composition en une teneur allant de 0,1 % à 95 % en poids, par rapport au poids total de la composition, de préférence allant de 1 % à 85 % en poids, et préférentiellement allant de 1 % à 80 % en poids.

La composition peut comprendre d'autres ingrédients (adjuvants) usuellement utilisés en cosmétique tels que les cires, les conservateurs, les actifs cosmétiques, les agents hydratants, les filtres UV, les épaississants, l'eau, les tensioactifs, les parfums.

Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.

La composition selon l'invention peut être une composition anhydre, c'est-à-dire une composition contenant moins de 2 % en poids d'eau, voire moins de 0,5 % d'eau, notamment exempte d'eau, l'eau n'étant pas ajoutée lors de la préparation de la composition mais correspondant à l'eau résiduelle apportée par les ingrédients mélangés.

La composition sous forme de poudre compacte peut être préparée en mélangeant les ingrédients de la phase pulvérulentes (particules d'organosilicone, charges et pigments) puis en ajoutant la phase grasse sous agitation, le mélange étant ensuite broyé, tamisé, puis versé dans une coupelle et compacté.

Le mélange de la phase pulvérulente et de la phase grasse broyé et tamisé est compacté à l'aide d'une presse, notamment en appliquant une pression allant de 0,5 MPa à 10 MPa, et de préférence allant de 1 MPa à 5 MPa.
La composition ainsi obtenue se présente sous forme de poudre compacte.

L'invention est illustrée plus en détails par les exemples décrits ci-après.

### Exemples 1 à 3 comparatifs :

On a préparé 3 poudres compactes selon l'invention (exemple 1) et deux poudres compactes ne faisant pas partie de l'invention (exemples 2 et 3) ayant les compositions suivantes :

Les teneurs sont exprimées en % en poids.

| | **Exemple 1** | **Exemple 2** | **Exemple 3** |
|---|---|---|---|
| Talc | 39,9 | 39,9 | 37,85 |
| Séricite | 33,25 | 33,25 | 31,5 |
| Mica | 4,75 | 4,75 | 4,5 |
| Dioxyde de titane | 5,2 | 5,2 | 4,9 |
| Oxydes de fer | 2,1 | 2,1 | 1,98 |
| Stéarate de zinc | 0,95 | 0,95 | 0,9 |
| Paraffine liquide | 3,8 | 3,8 | 8,6 |
| Phenyl triméthicone | 4,75 | 4,75 | 4,5 |
| Conservateurs | 0,3 | 0,3 | 0,27 |
| Particules(1) hémisphériques en forme de bol de largeur moyenne 2,5 µm, d'épaisseur 0,15 µm et de hauteur 1,2 µm, constituées de l'organosilicone* TAK-110 de la société Takemoto Oil & Fat | 5 | | 5 |
| Particules sphérique de polyméthylsilsesquioxane (Tospearl 145 B de GE Toshiba Silicone) | | 5 | |

| | | | |
|---|---|---|---|
| * polymère réticulé méthylsilanol/silicate (1) particules vendues sous la dénomination NLK-506 par la société Takemoto Oil & Fat | | | |

La composition de l'exemple 2 (hors invention) contient des particules de polyméthylsilsesquioxane à la place des particules d'organopolysiloxane réticulé présentes dans la composition de l'exemple 1 selon l'invention.

La composition de l'exemple 3 (hors invention) contient une quantité plus élevée d'huiles (liant liquide), soit 13,1 g, par rapport à la composition 1 qui contient 8,55 g d'huiles.

Chaque composition a été préparée en mélangeant l'ensemble des poudres puis en y ajoutant le liant (huiles), ce mélange étant ensuite broyé et tamisé jusqu'à obtention d'un mélange homogène. 14 g de ce mélange est placé dans une coupelle puis pressé sous une pression de 2 MPa.

Chaque composition a été testée par un panel d'expert constitué de 9 persones en évaluant les propriétés suivantes lors de la prise de la poudre compacte avec une éponge et son application sur la peau :
Quantité de produit pris avec une éponge (délitage)
Facilité d'étalement sur la peau (glissant)
Douceur a l'application
Couvrance du produit

Les notes attribuées par chaque personne vont de 0 a 3 :
3: excellent
2: satisfaisant
1: moyen
0: mauvais
On calcule la moyenne des notes attribuées.
Les notes moyennes obtenues sont les suivantes :

| | Ex 1 | Ex 2 | Ex 3 |
|---|---|---|---|
| Délitage | 3 | 1 | 0 |
| Glissant | 3 | 1 | 0 |
| Douceur | 2 | 2 | 1 |
| Couvrance | 2 | 1 | 0 |

On a constaté que seule la composition selon l'invention de l'exemple 1 permet d'obtenir les meilleures propriétés de délitage, de facilité d'application sur la peau, de douceur et de couvrance par rapport à ces mêmes propriétés obtenues avec les compositions de l'art antérieur (exemples 2 et 3).

## Revendications

1. Composition cosmétique comprenant une phase pulvérulente et une phase grasse liquide, la phase pulvérulente comprenant des pigments et des particules concaves sous forme de portions de sphères creuses constituées d'un matériau organosiliconé, les pigments étant présents en une teneur inférieure ou égale à 15 % en poids, par rapport au poids total de la composition, et la phase grasse liquide étant présente en une teneur inférieure ou égale à 13 % en poids.

2. Composition selon la revendication précédente, **caractérisée par le fait que** lesdites particules ont un diamètre moyen allant de 0,05 µm à 10 µm.

3. Composition selon la revendication précédente, **caractérisée par le fait que** les particules de portions de sphères creuses ont une section transversale en forme de fer à cheval ou d'arceau.

4. Composition selon l'une quelconque des revendications précédentes, **caractérisée par le fait que** le matériau organosiliconé est un polysiloxane réticulé de structure tridimensionnelle comprenant, ou constitué de, des motifs de formule (I) : SiO₂ et de formule (II) : R¹SiO_{1,5}
dans lesquelles R¹ désigne un groupe organique ayant un atome de carbone directement relié à l'atome de silicium.

5. Composition selon la revendication précédente, **caractérisée par le fait que** le groupe organique est un groupe organique réactif, un groupe organique non réactif, et de préférence un groupe organique non réactif.

6. Composition selon la revendication précédente, **caractérisée par le fait que** le groupe organique non réactif peut être un groupe alkyle en C₁-C₄, ou un groupe phényle.

7. Composition selon la revendication 5 ou 6, **caractérisée par le fait que** le groupe organique non réactif est un groupe méthyle.

8. Composition selon la revendication 5, **caractérisée par le fait que** le groupe organique réactif est choisi parmi un groupe époxy, un groupe (méth)acryloxy, un groupe alkényle, un groupe mercaptoalkyle, aminoalkyle, halogénoalkyle, un groupe glycéroxy, un groupe uréido, un groupe cyano.

9. Composition selon la revendication 5 ou 8, **caractérisée par le fait que** le groupe organique réactif est choisi parmi un groupe époxy, un groupe (méth)acryloxy, un groupe alkényle, un groupe mercaptoalkyle, aminoalkyle.

10. Composition selon la revendication 4, **caractérisée par le fait que** R¹ désigne un groupe méthyle.

11. Composition selon l'une quelconque des revendications précédentes, **caractérisée par le fait que** le matériau organosiliconé comprend les motifs (I) et (II) selon un rapport molaire motif (I) / motif (II) allant de 30/70 à 50/50, de préférence allant de 35/65 à 45/55.

12. Composition selon l'une quelconque des revendications précédentes, **caractérisée par** le fait les particules d'organosilicone sont susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

13. Composition selon la revendication précédente, **caractérisée par le fait que** dans l'étape (a), le rapport molaire du composé (III) au composé (IV) va de 30/70 à 50/50, de préférence va de 35/65 à 45/45, et préférentiellement est de 40/60.

14. Composition selon la revendication 12 ou 13, **caractérisée par le fait que** le rapport en poids de l'eau au total des composés (III) et (IV) va de 10/90 à 70/30 dans l'étape (a).

15. Composition selon la revendication 12, **caractérisée par le fait que** le groupe organique est un groupe organique réactif, un groupe organique non réactif, et de préférence un groupe organique réactif.

16. Composition selon la revendication 12, **caractérisée par le fait que** le groupe organique non réactif peut être un groupe alkyle en C₁-C₄, ou un groupe phényle.

17. Composition selon la revendication 12 ou 16, **caractérisée par le fait que** le groupe organique non réactif est un groupe méthyle.

18. Composition selon la revendication 15, **caractérisée par le fait que** le groupe organique réactif est choisi parmi un groupe époxy, un groupe (méth)acryloxy, un groupe alkényle, un groupe mercaptoalkyle, aminoalkyle, halogénoalkyle, un groupe glycéroxy, un groupe uréido, un groupe cyano.

19. Composition selon la revendication 15 ou 18, **caractérisée par le fait que** le groupe organique réactif est choisi parmi un groupe époxy, un groupe (méth)acryloxy, un groupe alkényle, un groupe mercaptoalkyle, aminoalkyle.

20. Composition selon la revendication 12, **caractérisée par le fait que** R¹ désigne un groupe méthyle.

21. Composition selon l'une quelconque des revendications 12 à 20, **caractérisée par le fait que** les catalyseurs d'hydrolyse et de polymérisation sont indépendamment choisis parmi l'hydroxyde de sodium, l'hydroxyde de potassium, le carbonate de sodium, l'hydrogénocarbonate de sodium, l'ammoniaque, la triméthylamine, la triéthylamine, l'hydroxyde de tétraméthylammonium, l'acide citrique, l'acide acétique, l'acide méthanesulfonique, l'acide p-toluène sulfonique, l'acide dodécylbenzènesulfonique, l'acide dodécylsulfonique , l'acide chlorhydrique, l'acide sulfurique, l'acide phosphorique.

22. Composition selon l'une quelconque des revendications précédentes, **caractérisée par le fait que** les particules concaves sont formées (en coupe longitudinale) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, dé préférence de 0,03 à 6 µm en moyenne.

23. Composition selon l'une quelconque des revendications précédentes, **caractérisée par le fait que** les portions de sphères creuses sont présentes en une teneur allant de 0,01 % à 50 % en poids, par rapport au poids total de la composition, de préférence allant de 0,1 % à 30 % en poids, et préférentiellement allant de 1 % à 15 % en poids.

24. Composition selon l'une quelconque des revendications précédentes, **caractérisée par le fait que** les pigments sont choisis parmi le dioxyde de titane, l'oxyde de zirconium, l'oxyde de cérium, les oxydes de zinc, les oxydes de fer , l'oxyde de chrome, le violet de manganèse, le bleu outremer, l'hydrate de chrome, le bleu ferrique, la poudre d'aluminium, la poudre de cuivre, le noir de carbone, les pigments de typé D & C, les laques.

25. Composition selon la revendication précédente, **caractérisée par le fait que** les pigments sont présents en une teneur allant de 0,1 % à 14,95 % en poids, par rapport au poids total de la composition, de préférence allant de 0,5 % à 12 % en poids, et préférentiellement allant de 1 % à 10 % en poids.

26. Composition selon l'une quelconque des revendications précédentes, **caractérisée par le fait qu'**elle comprend une matière colorante additionnelle choisie parmi les nacres, les paillettes.

27. Composition selon la revendication précédente, **caractérisée par le fait que** les nacres sont choisies parmi le mica recouvert de titane, ou d'oxychlorure de bismuth, le mica titane recouvert avec des oxydes de fer, le mica titane recouvert avec du bleu ferrique ou de l'oxyde de chrome, le mica titane recouvert avec un pigment organique du type précité, les pigments nacrés à base d'oxychlorure de bismuth.

28. Composition selon la revendication 26 ou 27, **caractérisée par le fait que** les nacres sont présentes en une teneur allant de 0,1 % à 50 % en poids, de préférence allant de 0,1 % à 40 % en poids, et préférentiellement allant de 0,1 % à 30 % en poids.

29. Composition selon l'une quelconque des revendications précédentes, **caractérisée par le fait que** la phase grasse liquide est présente en une teneur allant de 0,1 % à 13 % en poids, par rapport au poids total de la composition, de préférence allant de 0,1 % à 10 % en poids, et préférentiellement allant de 0,1 % à 8 % en poids.

30. Composition selon l'une quelconque des revendications précédentes, **caractérisée par le fait que** la phase grasse liquide comprend une huile choisie parmi l'huile de vison, l'huile de tortue, l'huile de soja, l'huile de pépins de raison, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide, les huiles de paraffine, le squalane, la vaseline, le polydécène, le myristate d'isopropyle, le palmitate d'isopropyle, le stéarate de butyle, le stéarate d'isodécyle, le laurate d'hexyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine, les huiles de silicone, les silicones fluorées, les huiles perfluorées, l'acide oléique, l'acide linoléique, l'acide linolénique, l'acide isostéarique, le cétanol, l'alcool oléique.

31. Composition selon l'une quelconque des revendications précédentes, **caractérisée par le fait qu'**elle comprend une charge.

32. Composition selon la revendication précédente, **caractérisée par le fait que** la charge est choisie parmi le talc, le mica, la silice, le kaolin, les poudres de polyamide, les poudres de poly-β-alanine, les poudres de polyéthylène, les poudres de polymères de tétrafluoroéthylène, la lauroyl-lysine, l'amidon, le nitrure de bore, les microsphères creuses polymériques, les microbilles de résine de silicone, les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthanes, les charges composites, les microsphères de silice creuses, les microcapsules de verre ou de céramique, les savons métalliques dérivés d'acides organiques carboxyliques ayant de 8 à 22 atomes de carbone.

33. Composition selon la revendication 31 ou 32, **caractérisée par le fait que** les charges sont présentes en une teneur allant de 0,1 % à 95 % en poids, par rapport au poids total de la composition, de préférence allant de 1 % à 85 % en poids, et préférentiellement allant de 1 % à 80 % en poids.

34. Composition selon l'une quelconque des revendications précédentes, **caractérisée par le fait qu'**elle comprend un ingrédient cosmétique choisi parmi les cires, les conservateurs, les actifs cosmétiques, les agents hydratants, les filtres UV, les épaississants, l'eau, les tensioactifs, les parfums.

35. Composition selon l'une quelconque des revendications précédentes, **caractérisée par le fait qu'**elle se présente sous forme de poudre compacte.

36. Composition selon l'une quelconque des revendications précédentes, **caractérisée par le fait que** la composition est sous forme d'un fard à joues, un fard à paupières, une poudre pour le visage, un fond de teint, un produit anticerne, un produit de maquillage du corps, un produit de soin pour le visage, un produit de soin du corps, un produit antisolaire.

37. Procédé cosmétique de maquillage ou de soin non thérapeutique des matières kératiniques d'être humain, en particulier de la peau, comprenant l'application sur les matières kératiniques, en particulier sur la peau, d'une composition selon l'une quelconque des revendications précédentes.

## Claims

1. Cosmetic composition comprising a pulverulent phase and a liquid fatty phase, the pulverulent phase comprising pigments and concave particles in the form of portions of hollow spheres composed of an organosilicone material, the pigments being present in a content of less than or equal to 15% by weight, with respect to the total weight of the composition, and the liquid fatty phase being present in a content of less than or equal to 13% by weight.

2. Composition according to the preceding claim, **characterized in that** the said particles have a mean diameter ranging from 0.05 µm to 10 µm.

3. Composition according to the preceding claim, **characterized in that** the particles of portions of hollow spheres have a transverse cross section with the shape of a horseshoe or arch.

4. Composition according to any one of the preceding claims, **characterized in that** the organosilicone material is a crosslinked polysiloxane with a three-dimensional structure comprising or composed of units of formula (I): SiO₂, and of formula (II): R¹SiO_{1.5},
in which R¹ denotes an organic group having a carbon atom directly connected to the silicon atom.

5. Composition according to the preceding claim,
**characterized in that** the organic group is a reactive organic group or an unreactive organic group and preferably an unreactive organic group.

6. Composition according to the preceding claim, **characterized in that** the unreactive organic group can be a C₁-C₄ alkyl group or a phenyl group.

7. Composition according to Claim 5 or 6,
**characterized in that** the unreactive organic group is a methyl group.

8. Composition according to Claim 5, **characterized in that** the reactive organic group is chosen from an epoxy group, a (meth)acryloyloxy group, an alkenyl group, a mercaptoalkyl, aminoalkyl or haloalkyl group, a glyceroxy group, a ureido group or a cyano group.

9. Composition according to Claim 5 or 8,
**characterized in that** the reactive organic group is chosen from an epoxy group, a (meth)acryloyloxy group, an alkenyl group or a mercaptoalkyl or aminoalkyl group.

10. Composition according to Claim 4, **characterized in that** R¹ denotes a methyl group.

11. Composition according to any one of the preceding claims, **characterized in that** the organosilicone material comprises the units (I) and (II) according to a unit (I)/unit (II) molar ratio ranging from 30/70 to 50/50, preferably ranging from 35/65 to 45/55.

12. Composition according to any one of the preceding claims, **characterized in that** the organosilicone particles are capable of being obtained according to a process comprising:
(a) the introduction into an aqueous medium, in the presence of at least one hydrolysis catalyst and optionally of at least one surfactant, of a compound (III) of formula SiX₄ and of a compound (IV) of formula RSiY₃, where X and Y denote, independently of one another, a C₁-C₄ alkoxy group, an alkoxyethoxy group including a C₁-C₄ alkoxy group, a C₂-C₄ acyloxy group, an N,N-dialkylamino group including a C₁-C₄ alkyl group, a hydroxyl group, a halogen atom or a hydrogen atom and R denotes an organic group comprising a carbon atom connected directly to the silicon atom; and
(b) the operation in which the mixture resulting from stage (a) is brought into contact with an aqueous solution including at least one polymerization catalyst and optionally at least one surfactant, at a temperature of between 30 and 85°C, for at least two hours.

13. Composition according to the preceding claim, **characterized in that,** in stage (a), the molar ratio of the compound (III) to the compound (IV) ranges from 30/70 to 50/50, preferably ranges from 35/65 to 45/45, and is preferentially 40/60.

14. Composition according to Claim 12 or 13,
**characterized in that** the ratio by weight of the water to the total of the compounds (III) and (IV) ranges from 10/90 to 70/30 in stage (a).

15. Composition according to Claim 12, **characterized in that** the organic group is a reactive organic group or an unreactive organic group and preferably a reactive organic group.

16. Composition according to Claim 12, **characterized in that** the unreactive organic group can be a C₁-C₄ alkyl group or a phenyl group.

17. Composition according to Claim 12 or 16,
**characterized in that** the unreactive organic group is a methyl group.

18. Composition according to Claim 15, **characterized in that** the reactive organic group is chosen from an epoxy group, a (meth)acryloyloxy group, an alkenyl group, a mercaptoalkyl, aminoalkyl or haloalkyl group, a glyceroxy group, a ureido group or a cyano group.

19. Composition according to Claim 15 or 18,
**characterized in that** the reactive organic group is chosen from an epoxy group, a (meth)acryloyloxy group, an alkenyl group or a mercaptoalkyl or aminoalkyl group.

20. Composition according to Claim 12, **characterized in that** R denotes a methyl group.

21. Composition according to any one of Claims 12 to 20, **characterized in that** the hydrolysis and polymerization catalysts are chosen independently from sodium hydroxide, potassium hydroxide, sodium carbonate, sodium hydrogencarbonate, ammonia, trimethylamine, triethylamine, tetramethylammonium hydroxide, citric acid, acetic acid, methanesulphonic acid, p-toluenesulphonic acid, dodecylbenzenesulphonic acid, dodecylsulphonic acid, hydrochloric acid, sulphuric acid or phosphoric acid.

22. Composition according to any one of the preceding claims, **characterized in that** the concave particles are formed (in longitudinal cross section) of a small internal arc (11), of a large external arc (21) and of segments (31) which connect the ends of the respective arcs, the width (W1) between the two ends of the small internal arc (11) ranging from 0.01 to 8 µm, preferably from 0.02 to 6 µm, on average, the width (W2) between the two ends of the large external arc (21) ranging from 0.05 to 10 µm, preferably from 0.06 to 8 µm, on average, and the height (H) of the large external arc (21) ranging from 0.015 to 8 µm, preferably from 0.03 to 6 µm, on average.

23. Composition according to any one of the preceding claims, **characterized in that** the portions of hollow spheres are present in a content ranging from 0.01% to 50% by weight, with respect to the total weight of the composition, preferably ranging from 0.1% to 30% by weight and preferentially ranging from 1% to 15% by weight.

24. Composition according to any one of the preceding claims, **characterized in that** the pigments are chosen from titanium dioxide, zirconium oxide, cerium oxide, zinc oxides, iron oxides, chromium oxide, manganese violet, ultramarine blue, chromium hydrate, ferric blue, aluminium powder, copper powder, carbon black, pigments of D & C type, or lakes.

25. Composition according to the preceding claim, **characterized in that** the pigments are present in a content ranging from 0.1% to 14.95% by weight, with respect to the total weight of the composition, preferably ranging from 0.5% to 12% by weight and preferentially ranging from 1% to 10% by weight.

26. Composition according to any one of the preceding claims, **characterized in that** it comprises an additional colouring material chosen from pearlescent agents or glitter.

27. Composition according to the preceding claim, **characterized in that** the pearlescent agents are chosen from mica covered with titanium or with bismuth oxychloride, titanium oxide-coated mica covered with iron oxides, titanium oxide-coated mica covered with ferric blue or chromium oxide or titanium oxide-coated mica covered with an organic pigment of the abovementioned type, and pearlescent pigments based on bismuth oxychloride.

28. Composition according to Claim 26 or 27,
**characterized in that** pearlescent agents are present in a content ranging from 0.1 to 50% by weight, preferably ranging from 0.1% to 40% by weight and preferentially ranging from 0.1% to 30% by weight.

29. Composition according to any one of the preceding claims, **characterized in that** the liquid fatty phase is present in a content ranging from 0.1% to 13% by weight, with respect to the total weight of the composition, preferably ranging from 0.1% to 10% by weight and preferentially ranging from 0.1% to 8% by weight.

30. Composition according to any one of the preceding claims, **characterized in that** the liquid fatty phase comprises an oil chosen from mink oil, turtle oil, soybean oil, grape seed oil, sesame oil, maize oil, rapeseed oil, sunflower oil, cottonseed oil, avocado oil, olive oil, castor oil, jojoba oil, groundnut oil, liquid paraffins, squalane, liquid petrolatum, polydecene, isopropyl myristate, isopropyl palmitate, butyl stearate, isodecyl stearate, hexyl laurate, isononyl isononanoate, 2-ethylhexyl palmitate, 2-hexyldecyl laurate, 2-octyldecyl palmitate, 2-octyldodecyl myristate, 2-octyldodecyl lactate, di(2-ethylhexyl) succinate, diisostearyl malate, glyceryl triisostearate, diglyceryl triisostearate, silicone oils, fluorinated silicones, perfluorinated oils, oleic acid, linoleic acid, linolenic acid, isostearic acid, cetanol or oleyl alcohol.

31. Composition according to any one of the preceding claims, **characterized in that** it comprises a filler.

32. Composition according to the preceding claim, **characterized in that** the filler is chosen from talc, mica, silica, kaolin, powders formed of polyamide, powders formed of poly-β-alanine, powders formed of polyethylene, powders formed of tetrafluoroethylene polymers, lauryllysine, starch, boron nitride, polymeric hollow microspheres, silicone resin microbeads, particles formed of polyorganosiloxane elastomers, precipitated calcium carbonate, magnesium carbonate, basic magnesium carbonate, hydroxyapatite, barium sulphate, aluminium oxides, polyurethane powders, composite fillers, hollow silica microspheres, glass or ceramic microcapsules, or metal soaps derived from organic carboxylic acids having from 8 to 22 carbon atoms.

33. Composition according to Claim 31 or 32,
**characterized in that** the fillers are present in a content ranging from 0.1% to 95% by weight, with respect to the total weight of the composition, preferably ranging from 1% to 85% by weight and preferentially ranging from 1% to 80% by weight.

34. Composition according to any one of the preceding claims, **characterized in that** it comprises a cosmetic ingredient chosen from waxes, preservatives, cosmetic active principles, moisturizing agents, UV screening agents, thickeners, water, surfactants or fragrances.

35. Composition according to any one of the preceding claims, **characterized in that** it is provided in the form of a compact powder.

36. Composition according to any one of the preceding claims, **characterized in that** the composition is in the form of a blusher, an eyeshadow, a face powder, a foundation, a concealer, a product for making up the body, a product for caring for the face, a product for caring for the body or an antisun product.

37. Cosmetic process for making up or for the non-therapeutic care of human keratinous substances, in particular the skin, comprising the application to the keratinous substances, in particular to the skin, of a composition according to any one of the preceding claims.

## Patentansprüche

1. Kosmetische Zusammensetzung, die eine pulverförmige Phase und eine flüssige Fettphase enthält, wobei die pulverförmige Phase Pigmente und konkave Partikel in Form von Teilen von hohlen Sphären umfasst, die aus einem Silicium-organischen Material gebildet werden, wobei die Pigmente in einer Menge von kleiner oder gleich 15 Gew.-%, bezogen auf das Gesamtgewicht der Zusammensetzung, enthalten sind und die flüssige Fettphase in einer Menge von kleiner oder gleich 13 Gew.-% vorliegt.

2. Zusammensetzung nach dem vorhergehenden Anspruch, **dadurch gekennzeichnet, dass** die Partikel einen mittleren Durchmesser von 0,05 bis 10 µm aufweisen.

3. Zusammensetzung nach dem vorhergehenden Anspruch, **dadurch gekennzeichnet, dass** die Partikel von Teilen von hohlen Sphären einen hufeisenförmigen Querschnitt oder einen bogenförmigen Querschnitt aufweisen.

4. Zusammensetzung nach einem der vorhergehenden Ansprüche, **dadurch gekennzeichnet, dass** das Silicium-organische Material ein vernetztes Polysiloxan mit dreidimensionaler Struktur ist, das Einheiten der Formel (I): SiO₂ und der Formel (II): R¹SiO_{1,5} enthält oder daraus besteht, wobei R¹ eine organische Gruppe bezeichnet, die ein Kohlenstoffatom aufweist, das direkt an das Siliciumatom gebunden ist.

5. Zusammensetzung nach dem vorhergehenden Anspruch, **dadurch gekennzeichnet, dass** es sich bei der organischen Gruppe um eine reaktive organische Gruppe, eine nichtreaktive organische Gruppe und vorzugsweise eine nichtreaktive organische Gruppe handelt.

6. Zusammensetzung nach dem vorhergehenden Anspruch, **dadurch gekennzeichnet, dass** die nichtreaktive organische Gruppe eine C₁₋₄-Alkylgruppe oder eine Phenylgruppe sein kann.

7. Zusammensetzung nach Anspruch 5 oder 6, **dadurch gekennzeichnet, dass** die nichtreaktive organische Gruppe die Methylgruppe ist.

8. Zusammensetzung nach Anspruch 5, **dadurch gekennzeichnet, dass** die reaktive organische Gruppe unter Epoxy, (Meth)acryloxy, Alkenyl, Mercaptoalkyl, Aminoalkyl, Halogenoalkyl, Glyceroxy, Ureido und Cyano ausgewählt ist.

9. Zusammensetzung nach Anspruch 5 oder 8, **dadurch gekennzeichnet, dass** die reaktive organische Gruppe unter einer Epoxygruppe, einer (Meth)acryloxygruppe, einer Alkenylgruppe, einer Mercaptoalkylgruppe oder einer Aminoalkylgruppe ausgewählt ist.

10. Zusammensetzung nach Anspruch 4, **dadurch gekennzeichnet, dass** R¹ die Methylgruppe bedeutet.

11. Zusammensetzung nach einem der vorhergehenden Ansprüche, **dadurch gekennzeichnet, dass** das Silicium-organische Material die Einheiten (I) und (II) in einem Molverhältnis Einheit (I)/Einheit (II) im Bereich von 30/70 bis 50/50 und vorzugsweise 35/65 bis 45/55 enthält.

12. Zusammensetzung nach einem der vorhergehenden Ansprüche, **dadurch gekennzeichnet, dass** die Silicium-organischen Partikel nach einem Verfahren erhältlich sind, das umfasst:
(a) Einbringen einer Verbindung (III) der Formel SiX₄ und einer Verbindung (IV) der Formel RSiY₃, wobei X und Y unabhängig voneinander eine C₁₋₄-Alkoxygruppe, eine Alkoxyethoxygruppe, die eine C₁₋₄-Alkoxygruppe umfasst, eine C₂₋₄-Acyloxygruppe, eine N,N-Dialkylaminogruppe, die eine C₁₋₄-Alkylgruppe umfasst, eine Hydroxygruppe, ein Halogenatom oder ein Wasserstoffatom bedeuten können und R eine organische Gruppe ist, die ein direkt an das Siliciumatom gebundenes Kohlenstoffatom umfasst, in ein wässriges Medium in Gegenwart mindestens eines Hydrolysekatalysators und gegebenenfalls mindestens eines grenzflächenaktiven Stoffes; und
(b) Inkontaktbringen des aus Schritt (a) resultierenden Gemisches mit einer wässrigen Lösung, die mindestens einen Polymerisationskatalysator und gegebenenfalls mindestens einen grenzflächenaktiven Stoff enthält, bei einer Temperatur im Bereich von 30 bis 85 °C während mindestens zwei Stunden.

13. Zusammensetzung nach dem vorhergehenden Anspruch, **dadurch gekennzeichnet, dass** in Schritt (a) das Molverhältnis der Verbindung (III) zu der Verbindung (IV) im Bereich von 30/70 bis 50/50 und vorzugsweise im Bereich von 35/65 bis 45/45 liegt und bevorzugt 40/60 ist.

14. Zusammensetzung nach Anspruch 12 oder 13, **dadurch gekennzeichnet, dass** das Gewichtsverhältnis von Wasser zu den gesamten Verbindungen (III) und (IV) in Schritt (a) im Bereich von 10/90 bis 70/30 liegt.

15. Zusammensetzung nach Anspruch 12, **dadurch gekennzeichnet, dass** die organische Gruppe eine reaktive organische Gruppe, eine nichtreaktive organische Gruppe und vorzugsweise eine reaktive organische Gruppe ist.

16. Zusammensetzung nach Anspruch 12, **dadurch gekennzeichnet, dass** die nichtreaktive organische Gruppe eine C₁₋₄-Alkylgruppe oder eine Phenylgruppe sein kann.

17. Zusammensetzung nach Anspruch 12 oder 16, **dadurch gekennzeichnet, dass** die nichtreaktive organische Gruppe die Methylgruppe ist.

18. Zusammensetzung nach Anspruch 15, **dadurch gekennzeichnet, dass** die reaktive organische Gruppe unter Epoxy, (Meth)acryloxy, Alkenyl, Mercaptoalkyl, Aminoalkyl, Halogenoalkyl, Glyceroxy, Ureido und Cyano ausgewählt ist.

19. Zusammensetzung nach Anspruch 15 oder 18, **dadurch gekennzeichnet, dass** die reaktive organische Gruppe unter einer Epoxygruppe, einer (Meth)acryloxygruppe, einer Alkenylgruppe, einer Mercaptoalkylgruppe und einer Aminoalkylgruppe ausgewählt ist.

20. Zusammensetzung nach Anspruch 12, **dadurch gekennzeichnet, dass** R¹ die Methylgruppe bedeutet.

21. Zusammensetzung nach einem der Ansprüche 12 bis 20, **dadurch gekennzeichnet, dass** der Hydrolysekatalysator und der Polymerisationskatalysator unabhängig voneinander unter Natriumhydroxid, Kaliumhydroxid, Natriumcarbonat, Natriumhydrogencarbonat, Ammoniak, Trimethylamin, Triethylamin, Tetramethylammoniumhydroxid, Citronensäure, Essigsäure, Methansulfonsäure, p-Toluolsulfonsäure, Dodecylbenzolsulfonsäure, Dodecylsulfonsäure, Salzsäure, Schwefelsäure und Phosphorsäure ausgewählt sind.

22. Zusammensetzung nach einem der vorhergehenden Ansprüche, **dadurch gekennzeichnet, dass** die konkaven Partikel (im Längsschnitt) aus einem inneren kleinen Bogen (11), einem äußeren großen Bogen (21) und Segmenten (31) gebildet werden, die jeweils die Enden der Bögen verbinden, wobei die Breite (W1) zwischen den beiden Enden des inneren kleinen Bogens (11) im Mittel im Bereich von 0,01 bis 8 µm und vorzugsweise 0,02 bis 6 µm liegt, die Breite (W2) zwischen den beiden Enden des großen äußeren Bogens (21) im Mittel im Bereich von 0,05 bis 10 µm und vorzugsweise 0,06 bis 8 µm liegt und die Höhe (H) des äußeren großen Bogens (11) im Mittel im Bereich von 0,015 bis 8 µm und vorzugsweise 0,03 bis 6 µm liegt.

23. Zusammensetzung nach einem der vorhergehenden Ansprüche, **dadurch gekennzeichnet, dass** die Teile der hohlen Sphären in einem Anteil von 0,01 bis 50 Gew.-%, bezogen auf das Gesamtgewicht der Zusammensetzung, vorzugsweise 0,1 bis 30 Gew.-% und noch bevorzugter 1 bis 15 Gew.-% enthalten sind.

24. Zusammensetzung nach einem der vorhergehenden Ansprüche, **dadurch gekennzeichnet, dass** die Pigmente unter Titandioxid, Zirconiumoxid, Ceroxid, Zinkoxiden, Eisenoxiden, Chromoxid, Manganviolett, Ultramarinblau, Chromhydrat, Eisenblau, Aluminiumpulver, Kupferpulver, Ruß, Pigmenten vom Typ D & C und Lacken ausgewählt sind.

25. Zusammensetzung nach dem vorhergehenden Anspruch, **dadurch gekennzeichnet, dass** die Pigmente in einer Menge von 0,1 bis 14,95 Gew.-%, bezogen auf das Gesamtgewicht der Zusammensetzung, vorzugsweise im Bereich von 0,5 bis 12 Gew.-% und noch bevorzugter im Bereich von 1 bis 10 Gew.-% enthalten sind.

26. Zusammensetzung nach einem der vorhergehenden Ansprüche, **dadurch gekennzeichnet, dass** sie ein ergänzenden Farbmittel enthält, das unter den Perlglanzstoffen und Pailletten ausgewählt ist.

27. Zusammensetzung nach dem vorhergehenden Anspruch, **dadurch gekennzeichnet, dass** die Perlglanzstoffe unter den mit Titan oder Bismutoxidchlorid bedeckten Glimmerpigmenten, mit Eisenoxiden bedeckten Titan-Glimmerpigmenten, mit Eisenblau oder Chromoxid bedeckten Titan-Glimmerpigmenten, mit einem organischen Pigment vom genannten Typ bedeckten Titan-Glimmerpigmenten und Perlglanzpigmenten auf der Basis von Bismutoxidchlorid ausgewählt sind.

28. Zusammensetzung nach Anspruch 26 oder 27, **dadurch gekennzeichnet, dass** die Perlglanzstoffe in einer Menge von 0,1 bis 50 Gew.-%, vorzugsweise 0,1 bis 40 Gew.-% und noch bevorzugter 0,1 bis 30 Gew.-% enthalten sind.

29. Zusammensetzung nach einem der vorhergehenden Ansprüche, **dadurch gekennzeichnet, dass** die flüssige Fettphase in einer Menge von 0,1 bis 13 Gew.-%, bezogen auf das Gesamtgewicht der Zusammensetzung, vorzugsweise 0,1 bis 10 Gew.-% und noch bevorzugter 0,1 bis 8 Gew.-% enthalten ist.

30. Zusammensetzung nach einem der vorhergehenden Ansprüche, **dadurch gekennzeichnet, dass** die flüssige Fettphase ein Öl enthält, das unter Nerzöl, Schildkrötenöl, Sojaöl, Traubenkernöl, Sesamöl, Maisöl, Rapsöl, Sonnenblumenöl, Baumwollsamenöl, Avocadoöl, Olivenöl, Ricinusöl, Jojobaöl, Erdnussöl, Paraffinölen, Squalan, Vaseline, Polydecen, Isopropylmyristat, Isopropylpalmitat, Butylstearat, Isodecylstearat,
Hexyllaurat, Isononylisononanoat, 2-Ethylhexylpalmitat, 2-Hexyldecyllaurat, 2-Octyldecylpalmitat, 2-Octyldodecylmyristat oder 2-Octyldodecyllactat, 2-Diethylhexylsuccinat, Diisostearylmalat, Glycerintriisostearat oder Diglycerintriisostearat, Siliconölen, fluorierten Ölen, perfluorierten Ölen, Ölsäure, Linolsäure, Linolensäure, Isostearinsäure, Cetanol und Oleylalkohol ausgewählt ist.

31. Zusammensetzung nach einem der vorhergehenden Ansprüche, **dadurch gekennzeichnet, dass** sie einen Füllstoff enthält.

32. Zusammensetzung nach dem vorhergehenden Anspruch, **dadurch gekennzeichnet, dass** der Füllstoff unter Talk, Glimmer, Kieselsäure, Kaolin, Polyamidpulvern, Poly-β-alaninpulvern, Polyethylenpulvern, Pulvern von Tetrafluorethylenpolymeren, Lauroyllysin, Stärke, Bornitrid, polymeren Mikrohohlkugeln, Siliconharzmikrokugeln, elastomeren Polyorganosiloxanpartikeln, gefälltem Calciumcarbonat, Magnesiumcarbonat, Magnesiumhydrogencarbonat, Hydroxyapatit, Bariumsulfat, Aluminiumoxiden, Polyurethanpulvern, zusammengesetzten Füllstoffen, Siliciumoxid-Mikrohohlkugeln, Mikrokapseln aus Glas oder Keramik, Metallseifen, die von organischen Carbonsäuren mit 8 bis 22 Kohlenstoffatomen abgeleitet sind, ausgewählt ist.

33. Zusammensetzung nach Anspruch 31 oder 32, **dadurch gekennzeichnet, dass** die Füllstoffe in einer Menge von 0,1 bis
95 Gew.-%, bezogen auf das Gesamtgewicht der Zusammensetzung, vorzugsweise 1 bis 85 Gew.-% und bevorzugt 1 bis 80 Gew.-% enthalten sind.

34. Zusammensetzung nach einem der vorhergehenden Ansprüche, **dadurch gekennzeichnet, dass** sie einen kosmetischen Bestandteil enthält, der unter den Wachsen, Konservierungsmitteln, kosmetischen Wirkstoffen, Hydratisierungsmitteln, UV-Filtern, Verdickungsmitteln, Wasser, grenzflächenaktiven Stoffen und Parfums ausgewählt ist.

35. Zusammensetzung nach einem der vorhergehenden Ansprüche, **dadurch gekennzeichnet, dass** sie in Form eines kompakten Puders vorliegt.

36. Zusammensetzung nach einem der vorhergehenden Ansprüche, **dadurch gekennzeichnet, dass** die Zusammensetzung als Wangenrouge, Lidschatten, Puder für das Gesicht, Make-up, Produkt gegen Augenringe, Produkt zum Schminken des Körpers, Produkt für die Pflege des Gesichts, Produkt für die Pflege des Körpers, Sonnenschutzprodukt vorliegt.

37. Kosmetisches Verfahren zum Schminken oder für die nichttherapeutische Pflege von menschlichen Keratinsubstanzen und insbesondere der Haut, das das Aufbringen einer Zusammensetzung nach einem der vorhergehenden Ansprüche auf die Keratinsubstanzen und besonders die Haut umfasst.
